# EUROPEAN PATENT SPECIFICATION

(11) **EP 1 740 708 B1**
(45) Date of publication and mention of the grant of the patent: **05.03.2014**
(21) Application number: 05734274.3
(22) Date of filing: 07.04.2005
(51) Int. Cl.: C12P 7/64, C12P 9/00

(54) **Enzymatic production of hydrolyzed lecithin products**
Enzymatische Herstellung von hydrolysierten Lecithinprodukten
Préparation enzymatíque de produits de lecithine hydrolysée

(30) Priority: 09.04.2004 US 822222
(43) Date of publication of application: 10.01.2007
(73) Proprietor: Cargill Incorporated, Wayzata MN 55391 (US)
(72) Inventor: SCHMITT, Heidi, 21521 Aumuehle (DE); HEIRMAN, Marc, 8500 Kortrijk (BE); BRÜSE, Falk, 59387 Ascheberg (DE); SCHNEIDER, Michael, 67251 Freinsheim (DE); VAN DER SYPE, John, 9890 Semmerzake (BE)
(74) Representative: Vossius & Partner
(86) International application number: PCT/US2005/011784
(87) International publication number: WO 2005/100579

(56) References cited:
- WO-A-00/52190
- WO-A-2005/024036
- PEARCE ET AL, NOVOZYMES A/S: "Lipases for food ingredients, Abstract 94-5" 2002, XP002337138 INTERNET ABSTRACT / 2002 ANNUAL MEETING AND FOOD EXPO - ANAHEIM, CALIFORNIA Retrieved from the Internet: URL:http://ift.confex.com/ift/2002/techpro gram/paper_10182.htm> [retrieved on 2005-07-20]

## Description

The invention pertains to processes for enzymatic production of hydrolyzed lecithin products in an aprotic organic solvent, such as hexane.

### REFERENCES

D. Adlercreutz et al., Synthesis of phosphatidylcholine with different fatty acid in sn-1 position by lipase-catalyzed esterification and transesterification reaction. Biotechnol. Bioeng. 78: 403-411 (2002)
A. Bastida et al., A single step purification, immobilization, and hyperactivation of lipases via interfacial adsorption on strongly hydrophobic supports. Biotechnology and Bioengineering 58(5):486-493 (1998)
V.M. Balcao et al., Bioreactors with immobilized lipases: State of the art. Enzyme and Microbial Technology 18:391-416 (1995).
G.F. Bickerstaff, ed., Immobilization of Enzymes and Cells. Humana, Totawa, NJ, 1997.
U.T. Bornscheuer et al., Optimizing lipases and related enzymes for efficient application. Trends in Biotechnology 20(10):433-437 (2002).
O. Chmiel *et al.,* Process for the interesterification of phospholipids. U.S. Patent No. 5,989,599 (1999).
W. Cho et al., Efficient immobilization of phospholipase A2. Methods in Molecular Biology 109:303-307 (1999).
R Fernandez-Lafuente et al., Immobilization of lipases by selective adsorption on hydrophobic supports. Chemistry and Physics of Lipids 93(1-2):185-97 (Jun 1998).
M.J. Haas et al., The hydrolysis of phosphatidylcholine by an immobilized lipase: optimization of hydrolysis in organic solvents. Journal of the American Oil Chemists' Society 70(2):111-17 (1993).
M. Haas et al., Enzymic phosphatidylcholine hydrolysis in organic solvents: an examination of selected commercially available lipases. Journal of the American Oil Chemists' Society 71(5):483-90 (1994).
M.J. Haas et al., The enzymic hydrolysis of triglyceride-phospholipid mixtures in an organic solvent. Journal of the American Oil Chemists' Society 72(5):519-25 (1995).
F. Hara et al., Comparative study of commercially available lipases in hydrolysis reaction of phosphatidylcholine. Journal of the American Oil Chemists' Society 74(9):1129-1132 (1997).
F.D. Gunstone, Enzymes as biocatalysts in the modification of natural lipids. Journal of the Science of Food and Agriculture 79:1535-1549 (1999).
A.E. Ivanov et al., Methods for the immobilization of lipases and their use for ester synthesis. Journal, of Molecular Catalysis B: Enzymatic 3:303-309 (1997).
B. Jirjis *et al.,* Method for removing phospholipids from vegetable oil miscella, method for conditioning a polymeric microfiltration membrane, and membrane. U.S. Patent No. 6,207,209 (2001).
B. Jirjis *et al.,* Method and apparatus for processing vegetable oil miscella, method for conditioning a polymeric microfiltration membrane, membrane, and lecithin product. U.S. Patent No. 6,833,149 (2004).
S.T. Kang et al., Characteristics of immobilized lipase-catalyzed hydrolysis of olive oil of high concentration in reverse phase system. Biotechnology and Bioengineering 33(11):1469 -76 (1989).
F.X. Malcata et al,. Kinetics and mechanisms of reactions catalysed by immobilized lipases. Enzyme and Microbial Technology 14(6):426-46 (Jun 1992).
F.X. Malcata et al., Immobilized lipase reactors for modification of fats and oils - a review. Journal of the American Oil Chemists Society 67(12):890-910 (1990).
K. Mosbach, ed., Methods in Enzymology, Vol. 137: Immobilized Enzymes and Cells. Academic Press, San Diego, CA, 1988.
A. Mustranta et al., Comparison of lipases and phospholipases in the hydrolysis of phospholipids. Process Biochemistry 30(5):393-401 (1995).
I.C. Omar et al., Hydrolysis of triglycerides by immobilized thermostable lipase from Humicola lanuginosa. Agricultural and Biological Chemistry 52(1):99-105 (1988).
M.V. Ramachandra et al., Hydrolysis of oils by using immobilized lipase enzyme: A review. Biotechnology and Bioprocess Engineering 7(2):57-66 (2002).
B. Sas *et al.,* Improved method for the conversion of lecithin into lysolecithin. PCT Pubn. No. WO 00/52190 (2000).
J.F. Shaw et al., Lipolytic activities of a lipase immobilized on six selected supporting materials. Biotechnology and Bioengineering 35(2):132-7 (1990).
D.K. Shen et al., Characterisation and expression of phospholipases B from the opportunistic fungus Aspergillus fumigatus. FEMS Micro biology Letters 239(1):87-93 (2004).
S.W. Pearce et al., Lipases for food ingredients. Abstract 94-5, 2002 Annual Meeting and Food Expo, Anaheim, California

Lecithins and modified lecithins are widely used in industry, including the food and pharmaceutical industries, as solubilizers, emulsifiers, and other types of additives. Lecithins are obtained from various animal or vegetable sources, such as soybeans or egg yolk, and comprise a mixture of phospholipids and triglycerides, as well as lesser amounts of compounds such as glycolipids, carbohydrates, fatty acids, and/or sterols.

Partial hydrolysis of phospholipids in lecithins has been found to improve emulsifying properties. This modification is most commonly effected by treatment of lecithin with phospholipase A1 and/or A2, which selectively hydrolyze the first or second glyceryl fatty acid, respectively, of phospholipids, producing lysophospholipids.

Addition of mono- and diglycerides to lecithins or partially hydrolyzed lecithins has also been found to improve properties in end products, such as baking characteristics in flour or antispattering properties of margarine, especially those having a lower fat content, *e.g.* 60 wt. % fat or below (see *e.g.* EP Patent No. 253429; U.S. Patent No. 3,505,074; PCT Pubn. No. WO 02/49444). Further examples of food and feed products in which such compositions are useful include frozen dough, bakery products, to improve antistaling properties, emulsified meat products, ice cream, dressings, and other emulsion systems.
S.W. Pearce et al. disclose the use of a phospholipase, Lecitase, for hydrolyzing lecithin.

Currently, lecithin products containing enhanced quantities of mono- and diglycerides are prepared by adding these components to lecithins. It would be useful to prepare such products directly, in a controlled manner, by enzymatic hydrolysis of lecithin and related compositions.

### I. Overview

In one aspect, the invention provides a method of producing a hydrolyzed lecithin product comprising hydrolyzed phospholipids, monoglycerides, and diglycerides, the method comprising:
(a) contacting a lecithin material, which comprises a phospholipid component and a triglyceride component, with a first enzyme to create a reaction mixture in an organic solvent medium comprising an aprotic organic solvent and sufficient water to promote hydrolysis, wherein the first enzyme comprises a phospholipase or lipase which hydrolyzes the phospholipids;
(b) subsequently contacting the product of step (a), in an organic solvent medium containing sufficient water to promote hydrolysis, with a second enzyme that comprises a lipase which hydrolyzes the triglycerides and is different than the first enzyme; and
(c) obtaining the hydrolyzed lecithin product.

In selected embodiments, the second enzyme is a lipase, and the first enzyme is a phospholipase, *e.g*. phospholipase A1 and/or A2. In one such embodiment, the first enzyme is phospholipase A2.

In another embodiment, the first enzyme is phospholipase D. This embodiment may further comprise, prior to said contacting step (b), reacting the product of step (a) with phospholipase A1 and/or A2. In still another embodiment, the first enzyme is phospholipase C.

In this process, and all of the hydrolysis processes provided herein, the starting lecithin material is preferably derived from a naturally occurring source, such as egg yolk or a vegetable oil. Preferably, the starting material is derived directly from the naturally occurring source, i.e. without addition of external phospholipid, triglyceride, mono/diglyceride, or fatty acid components.

The starting material may be a lecithin material having an acetone insoluble (A.I.) content of at least 50%, preferably at least 60%, by weight. Proportions described herein are weight percents unless otherwise indicated. Alternatively, starting materials having lower values of A.I., *e.g.* 10%-50% by weight, may be used.

The hydrolyzed product is preferably a hydrolyzed lecithin product having at least 50%, and more preferably at least 56%, acetone insoluble materials. The hydrolyzed product preferably has an acid value of less than 45 mg KOH/gram. In further embodiments, the hydrolyzed product comprises at least 60% acetone insoluble materials.

The aprotic organic solvent may comprise a hydrocarbon solvent. The starting material may be a retentate from a vegetable oil membrane degumming process. In one embodiment of this aspect, steps (a) and (b) are carried out in the presence of a membrane effective to separate the hydrolyzed phospholipids, monoglycerides, and diglycerides from released fatty acids.

The starting material may be contacted with the first and second enzymes simultaneously or the enzymes may be employed sequentially, in either order. That is, the "first" enzyme may be employed before, after, or concurrently with the "second" enzyme.

Preferably, the first enzyme (i.e., the enzyme effective to hydrolyze the phospholipid) is phospholipase A1 and/or A2. In one embodiment, the first enzyme is phospholipase A2.

The aprotic organic solvent may be a hydrocarbon solvent, such as hexane. In one embodiment, the lecithin starting material is a retentate from a vegetable oil membrane degumming process.

In one embodiment, the enzyme is a lipase, and the hydrolyzed product comprises phospholipids and diglycerides and, in some cases, monoglycerides. In another embodiment, the enzyme is phospholipase C, and the hydrolyze product comprises phospholipids, triglycerides and diglycerides. In this case, the method may further comprise reacting the hydrolyzed product with phospholipase A1 and/or A2.

Each of the processes disclosed herein preferably comprise the further steps of removing or deactivating the enzyme(s), and removing the solvent medium. These steps may be further followed by formulation of the hydrolyzed product into a food product.

Further described is a hydrolyzed lecithin product, wherein the product contains hydrolyzed phospholipids, monoglycerides, and diglycerides, and is produced by any of the processes disclosed above, where the starting material is directly derived from a vegetable oil, and the process includes the steps of removing or deactivating the enzyme(s) and removing the solvent medium. The hydrolyzed lecithin product consists essentially of components of the starting material and hydrolyzed substances which are obtained from the starting material by reaction of the enzyme(s). Preferably, the hydrolyzed lecithin product contains at least 50%, and more preferably at least 56%, acetone insoluble materials and has an acid value of less than 45 mg KOH/gram. Most preferably, the product contains at least 60% acetone insoluble materials.

Also described is a food product comprising the hydrolyzed lecithin product and a method of formulating a food product, by combining a hydrolyzed lecithin product with additional consumable ingredients.

### II. Processes for Enzymatic Modification of Lecithin or Related Compositions

The invention provides methods for producing a hydrolyzed lecithin product, comprising phospholipids and/or hydrolyzed phospholipids, monoglycerides, and diglycerides (the last two components referred to jointly as mono/diglycerides). Typically, the product also includes unhydrolyzed phospholipids and triglycerides. The hydrolyzed phospholipids are typically lysophospholipids, and may also include phosphatidic acid and/or lysophosphatidic acid.

In accordance with the invention, the hydrolyzed product is produced directly by enzymatic treatment of a starting material, as described below, without separate addition of any of the above named components. The starting lecithin material is preferably derived directly from a vegetable oil; e.g. a material obtained from conventional processing of a vegetable oil.

In preferred embodiments, the process produces a hydrolyzed lecithin pro duct, comprising at least 50%, preferably at least 56%, and more preferably at least 60%, acetone insoluble materials (which consist primarily of phospholipids and/or hydrolyzed phospholipids) and having an acid value of less than 45 mg KOH/gram.

### A. Starting Materials

The processes disclosed herein employ a starting lecithin material having a phospholipid component (preferably at least 10%, and more preferably at least 25%, and most preferably at least 40% by weight) and a triglyceride component. When the starting material comprises a level of acetone insoluble materials (which consist primarily of phospholipids) which comports with legislative standards for "lecithin", the starting material may be referred to as a lecithin starting material This level of acetone insolubles is generally at least 50% by weight; e.g. 56% or more by weight, or 60% or more by weight.

The starting material is preferably derived from a naturally occurring source, such as egg yolk or a vegetable oil, where a "vegetable oil" includes any oil extracted from the seeds, fruit or nuts of a plant. Common examples include sunflower, rapeseed, canola, cottonseed, and, in particular, soybean oil. Preferably, the starting material is derived directly from the naturally occurring source, i.e. without addition of external phospholipid, triglyceride, mono/diglyceride, or fatty acid components.

Conveniently, the starting material may be produced via conventional pro cessing of naturally occurring materials, typically a vegetable oil, such as soybean oil. In preferred embodiments, the starting material is a material obtained by water or membrane degumming of a vegetable oil.

For example, in a typical procedure for processing of crude soybean oil, soybeans are dehulled and extracted with hexane to produce an extractant (miscella) which includes hexane and crude soybean oil. The crude soybean oil contains, as a major component, glyceride oil, in addition to phospholipids, sugars, sterols, sterol glucosides, fatty acids, and other components in minor amounts.

Phospholipids are separated from the majority of the glyceride oil in the miscella in a process known as "degumming". In conventional water degumming, this is generally done by removing the hexane solvent, hydrating the phospholipids with hot water or steam, and centrifuging. Removal of some or all of the water gives a crude lecithin material, typically containing about 60-65% acetone insolubles, with the remainder primarily triglycerides, and minor amounts of other components, such as sugars, sterol glucosides, and/or fatty acids. Such material may be used as a starting material in the processes described herein.

More recently, methods have been developed for membrane degumming of soybean oil, which generally produces a purer product than water degumming. See, for example, Jirjis et al., U.S. Patent Nos. 6,207,209 and 6,833,149. In a preferred embodiment of the present invention, the starting material is provided by such a process. In membrane degumming, a vegetable oil miscella in a hydrocarbon solvent, typically hexane, is fed to a membrane, producing a permeate stream which is depleted in phospholipids and a retentate stream which is enriched in phospholipids, relative to the phospholipid content of the miscella.

The retentate, with or without the hexane solvent, can be used as a starting material in the present processes. The phospholipid content of such a retentate can be varied by varying the separation conditions, *e.g.* the retentate-permeate ratio, separation time, and/or number of separation stages. (See *e.g.* Jirjis *et al.,* cited above.) The phospholipid content of the retentate (exclusive of solvent) can be, for example, between about 10% and about 85%, and is typically between about 50%-85%.

### B. Reaction Conditions: General

The reaction can be carried out in an organic solvent medium. As used herein, the term "organic solvent medium" refers to an aprotic solvent (*i.e.* not an alcohol or amine), and more preferably a nonpolar solvent, whereby the reaction medium includes sufficient water content for hydrolysis to occur. Preferably, the water content is at a level effective to promote hydrolysis and minimize transesterification. This water may be added to the reaction medium, *e.g.* by using a water saturated solvent, or it may be provided by entrapped or residual water in the starting material

In selected embodiments, the hydrolysis processes as described herein are carried out under conditions effective to inhibit transesterification of hydrolyzed phospholipids with released fatty acids. Such conditions may include the presence of sufficient water to inhibit esterification, or the presence of salts to sequester released fatty acids. For example, a salt or weak base may be added to the reaction mixture to sequester the released fatty acids. Such a salt may be, for example, excess calcium chloride. Calcium chloride is routinely included as an ionic activator in reactions employing phospholipases.

Alternatively or in addition, the reaction may be carried out in the presence of a membrane having a composition and pore size effective to remove fatty acids from the reaction mixture. This feature also serves to increase the acetone insolubles (A.I.) content of the hydrolyzed product.

Other reaction conditions that can be varied in the processes described herein include, for example, state of enzyme (*e.g.* whether immobilized or not), temperature, pH, presence and concentration of other additives, and substrate concentration. Exemplary conditions are described below.

### C. Two-Enzyme Hydrolysis Processes

The starting material is hydrolyzed in a two-enzyme process, using two different enzymes. Specifically, a first enzyme, typically a phospholipase (or a phospholipid reactive lipase, as discussed below) is used to hydrolyze phospholipids in the starting material to lysophospholipids and/or phosphatidic acids, and a second enzyme, a lipase (or, in some cases, a phospholipase having lipase activity) is used to hydrolyze triglycerides to mono/diglycerides. Preferably, the first enzyme is a phospholipase, such as phospholipase A2, for hydrolysis of phospholipids, and the second enzyme is a lipase, for hydrolysis of triglycerides. Note that the use of "first" and "second" in this case does not necessarily indicate chronological order of reaction.

The use of two different enzymes can provide greater flexibility in the ranges of product compositions obtained, with respect to the relative amounts of the different hydrolyzed components present, than a single enzyme process. The two enzymes can be used simultaneously or sequentially, in either order, as described further below.

More generally, each of the two different enzymes is a phospholipase or a lipase, where one enzyme is provided to hydrolyze phospholipids in the starting material, *e.g.* to lysophospholipids, and the other is provided to hydrolyze triglycerides in the starting material to mono/diglycerides. Typically, one enzyme is a phospholipase that selectively hydrolyzes phospholipids, and the other is a lipase that selectively hydrolyzes triglycerides. "Selective" as used herein indicates that the enzyme hydrolyzes a particular substrate, such as a phospholipid, to a greater degree than it hydrolyzes a different substrate, such as a triglyceride, under equivalent conditions. "Degree" of hydrolysis refers to mole percent of substrate hydrolyzed. The degree of hydrolysis of one substrate relative to another by a selective enzyme may differ by a factor of two or more, by a factor of about 5 or more, or by a factor of about 25 or about 50 or more.

As discussed below, some lipases are known to hydrolyze phospholipids, in some cases to a greater degree than they hydrolyze triglycerides. Conversely, though less commonly, some phospholipases are known to hydrolyze triglycerides, in some cases to a greater degree than they hydrolyze phospholipids. Generally, the activity and selectively of such enzymes is dependent on reaction conditions.

Accordingly, the selection of the two enzymes may include different combinations of lipase(s) and phospholipase(s), provided that one has significant and/or selective lipase activity and the other has significant and/or selective phospholipase activity, under the conditions of the reaction. "Significant" activity as used herein indicates that an enzyme hydrolyzes at least 1 mole %, preferably at least 5%, and more preferably at least 10% or at least 25 mole % of a particular substrate, under a given set of reaction conditions.

As described further below, the two-enzyme reaction, when carried out in aqueous solvent, advantageously employs a phospholipase (or phospholipid-selective) enzyme in a first stage, and a lipase (or triglyceride-selective) enzyme in a later stage. Reaction in organic solvent can employ the two enzymes sequentially, in either order, or simultaneously. The latter reaction is preferably performed on a retentate from a membrane degumming process.

### C. Two-Enzyme Reaction in Organic Solvent

The two-enzyme process is carried out in an aprotic organic solvent medium, containing sufficient water for hydrolysis to take place, and preferably sufficient water to promote hydrolysis and inhibit transesterification. The starting material is preferably provided in the organic solvent. In one embodiment, the process is carried out on a retentate obtained in membrane degumming of vegetable oil, typically soybean oil. A typical retentate, as described, for example, in U.S. Patent Nos. 6,207,209 and 6,833,149, contains the majority of the phospholipids present in the preprocessed oil, since these compounds form large aggregates in the hydrocarbon solvent which do not penetrate the membrane pores. However, as noted above, retentates having varying levels of phospholipids can be obtained, by varying the conditions used during membrane separation; *e.g*. by varying the retentate-permeate ratio. The retentate generally contains a significant fraction of triglycerides as well.

The aprotic organic solvent can be any solvent which is inert to the reaction conditions, including, for example, low molecular weight esters or ketones, halogenated hydrocarbons, or, preferably, hydrocarbons. Preferred hydrocarbon solvents include alkanes, cycloalkanes, and simple aromatic hydrocarbons, e.g., benzene and its homologs containing alkyl substituents having up to four carbon atoms. Exemplary hydrocarbons include benzene, toluene, xylenes, C₃-C₆ cycloalkanes, C₅-C₈ alkanes, mixtures thereof, *e.g*. petroleum ether, and C₅-C₈ alkenes.

When the two-enzyme process is carried out in an organic solvent medium, the two enzymes may be used simultaneously. Examples of reactions are provided below in Sections V and VI.

The process can be carried out in the presence of a membrane effective to separate the components of the hydrolyzed product, *e.g.* phospholipids, hydrolyzed phospholipids, triglycerides, monoglycerides, and/or diglycerides, from released fatty acids. A suitable membrane composition, pore size, and operating pressure can be selected, according to methods known in the art, to allow the free fatty acids to selectively pass through the membrane. This feature can be effective to increase the acetone insoluble (A.I.) content of the hydrolyzed product.

As discussed further below, the enzymes may be immobilized, which facilitates their removal from the reaction mixture. Non-immobilized enzymes are deactivated following completion of the reaction, typically by heating.

### D. Single-Enzyme Processes

In another embodiment of the invention, a retentate from a vegetable oil membrane degumming process, as described above, is used as the starting material in a single-enzyme hydrolysis process, where the enzyme is a phospholipase or, preferably, a lipase. As described above, and further in Section III below, many lipases have significant phospholipase activity and are effective to hydrolyze both triglycerides (to mono/diglycerides) and phospholipids (to lysophospholipids). The ratio of these activities in an enzyme preparation often varies with reaction conditions, particularly solvent and pH. Preferably, the lipase is triglyceride selective, as defined herein.

When the enzyme is a lipase, the hydrolyzed product, which may be a hydrolyzed lecithin product, having 50% or greater A.I., typically contains mono/diglycerides, triglycerides, and phospholipids, and may also contain hydrolyzed phospholipids, *e.g.* lysophospholipids. Examples of such reactions are given below in Sections V and VI.

Also contemplated are single-enzyme reactions where the enzyme is a phospholipase C. Again, the starting material may be derived from processing of vegetable oil, especially soybean oil, and is preferably derived directly from such a source. The reaction is expected to produce a product containing phospholipids, triglycerides and diglycerides, with no significant amount of monoglycerides or lysophospholipids. This product could be further reacted, if desired, with a phospholipase A, to produce a product further containing lysophospholipids.

### III. Enzymes Suitable for Use in the Invention Processes

### A. Phospholipases

As described above, the methods of the invention may employ a phospholipase to hydrolyze phospholipids (and, in some cases, triglycerides) in the starting material. Phospholipase enzymes are readily available commercially. Common phospholipases are categorized as A1, A2, C, and D on the basis of which bond in a phospholipid is hydrolyzed by the enzyme, as indicated below. (The phospholipase B family of enzymes share phospholipase, lysophospholipase and lysophospholipase-transacylase activities (Shen *et al.,* 2004) and are generally less preferred due to lower selectivity.)

The present two-enzyme processes typically employ phospholipase A1 (EC 3.1.1.32) and/or A2 (EC 3.1.1.4), which produces lysophospholipids by removal of an acyl side chain. In other embodiments, a phospholipase D (EC 3.1.4.4) may be us ed, alone or in combination with phospholipase A1 or A2, to provide phosphatidic acids and/or lysophosphatidic acids.

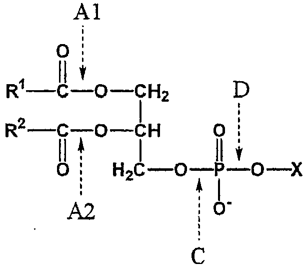

In a further embodiment, phospholipase C can be reacted with the phospholipid/triglyceride starting material to produce a product containing phospholipids, triglycerides and diglycerides, with no significant amount of monoglycerides or lysophospholipids. This product could be further reacted, if desired, with a phospholipase A, to produce a product further containing lysophospholipids.

In selected embodiments, phospholipase D is used alone or in combination with phospholipase A1 or A2 to produce hydrolyzed lecithin products having a phosphatidic acid or lysophosphatidic acid component. It may also be used in combination with an alcohol selected from ethanolamine, serine, and inositol, to increase, respectively, the PE, PS, or PI content of the hydrolyzed lecithin product.

Hydrolysis of lecithins with phospholipases A1 or A2 alone is known in the art. See, for example, GB Patent No. 1215868 (1970), which describes a conventional process in which lecithin is reacted with phospholipase A2 in aqueous emulsion, in the presence of calcium ions, to provide lysolecithin. Purification includes extraction with acetone, which removes acetone soluble materials such as fatty acids and glycerides. Hirai et al. (U.S. Patent No. 5,955,327) describes reaction of hydrated lecithin with phospholipase A1 or A2 in aqueous medium, followed by partitioning with acetone to isolate the lysolecithin product. Yesair (U.S. Patent No. 5,716,814) describes a process in which a mixture of phosphatidyl choline and a monoglyceride are reacted with phospholipase A2, to yield a lysophospholipid-monoglyceride-fatty acid composition.

In some cases, as discussed above, a phospholipase may exhibit significant and/or selective lipase activity. For example, Mustranta *et al.,* 1995, cited above, reported that a phospholipase A1 preparation *(A. niger*) had a 2:1 lipase/phospholipase activity ratio in aqueous medium. Such a phospholipase can be used to hydrolyze triglycerides in the processes described herein. More typically, however, a lipase, such as described below, is used to hydrolyze triglycerides.

### B. Lipases

Many different lipases (also referred to as triacylglycerol hydrolases; EC 3.1.1.3), obtained from animal, plant, or microbial sources, are known and/or commercially available, often in immobilized form. These enzymes can vary widely in activity and selectivity; that is, in the degree of lipase activity, phospholipase activity, and/or esterase activity exhibited by a particular enzyme preparation.

Information regarding activity and selectivity of a commercial lipase preparation will often be provided by the manufacturer. Enzyme activity and selectivity often varies with reaction conditions, e.g. whether the reaction is done in organic or aqueous medium, whether the enzyme is immobilized, *etc.,* and with the purity of the particular preparation. Other parameters than can affect reactivity and selectivity of an enzyme include pH, substrate concentration, and solvent polarity (see *e.g.* Haas *et al.,* 1995, 1994; cited above).

The activity of a particular enzyme preparation with respect to different substrates, such as phospholipids and triglycerides, can be evaluated empirically for a given set of reaction conditions, if desired, using methods known in the art. For example, A. Mustranta *et al.* (cited above) determined the degree of hydrolysis of soy phosphatidyl choline (*i.e.,* phospholipase activity) for each of various lipases and phospholipases, in aqueous emulsion, by titration of released fatty acids produced by 30 minutes of reaction. Similar reaction with olive oil was used to determine lipase activity. In this report, anA. *niger* lipase and a *P. cyclopium* lipase both showed phospholipase activity, though lipase activity was higher, particularly for the *A. niger* lipase. The report also found the phospholipase A2 preparations to be much more phospholipase selective than the phospholipase A1 preparations.

M. Haas *et al.* (1995, cited above) determined the degree of hydrolysis of phospholipids and triglycerides by each of three lipases and one phospholipase, each commercially available in immobilized form. The components were treated with the enzymes separately in water; mixtures of the components were treated in water-saturated hexane. Degree of hydrolysis was determined by titration of released fatty acids and by HPLC analysis of the product lipid mixtures. Solvent medium was found to have a large effect on reactivity and selectivity in this report. For example, lipases from *R. miehei* and *C. rugosa* were much more reactive towards triglycerides than towards phospholipids in both solvent media, and a third lipase, from *C. antarctica,* showed similar selectivity to *R. miehei* in water but was unreactive in hexane. The phospholipase (Amano phospholipase B) hydrolyzed only the phospholipids in hexane and was unreactive in water.

In another analysis, F. Hara *et al.* (cited above) determined the degree of hydrolysis of phosphatidyl choline by various lipases in a reverse micellar system over 24 hrs and over 48 hrs. The degree of hydrolysis, determined by chromatographic analysis, varied from 0 to 100% for the lipases tested. For example, under the reaction conditions described, lipases derived from *M. javanicus* (Lipase M10, Amano Pharmaceuticals), *M. miehei* (Lipozyme IM20, Novo Nordisk), and hog pancreas (Pancreatin F, Amano) gave 100% hydrolysis of phosphatidyl choline; lipases derived from *Rhizopus* sp. (Lipase F, Amano) and *R. delemar* (Newlase F, Amano) gave about 35-45% hydrolysis; and lipases derived from *A. niger* (Lipase A6, Amano) and *C. cylindrica* (Lipase AY30, Amano) gave little or no hydrolysis.

As shown in the above-cited articles and others in the field, lipases often exhibit significant phospholipase activity. Such enzymes, which may be referred to as "phospholipid reactive" lipases herein, may be used as the first enzyme in the two-enzyme processes described herein. A lipase which is "phospholipid reactive" may be defined as one which, under suitable reaction conditions such as those set forth in the exemplary reactions below, is effective to hydrolyze at least 1 mole %, preferably at least 5%, and more preferably at least 10% or at least 25 mole % of phospholipids present in the reaction. Such reaction conditions may include reaction in an aqueous medium, employing 0.001 to 0.2% enzyme based on 60% AI (acetone insolubles), at about 40-60°C for 4 to 24 hours, or reaction in an organic solvent, employing the same level of enzyme, at about 20-60°C for 1 to 24 hours.

More typically, the first enzyme in the two-enzyme reaction is a phospholipase, and lipases are employed as the second enzyme in the two-enzyme processes described herein, or as the single enzyme in processes targeting selective hydrolysis of the triglyceride component of the starting material. For these purposes, preferred lipases can be defined as those which, under the given reaction conditions, are selective for lipase activity; that is, the degree of hydrolysis of acyl glycerols by the lipase is greater, preferably by a factor of two or more, than the degree of hydrolysis of acyl phospholipids by the lipase, under the conditions of the reaction. (As noted above, reaction conditions include, for example, solvent, state of enzyme (*e.g.* whether immobilized or not), temperature, pH, presence and concentration of ionic additives, and substrate concentration.) "Degree of hydrolysis" can be defined as the mole percent of substrate hydrolyzed; *i.e.* percent of triglycerides converted to mono/diglycerides, or percent of phospholipids converted to lysophospholipids. More preferably, the degree is about 5 times greater, most preferably about 25 or about 50 times greater. These enzymes are also referred to herein as "triglyceride-selective" lipases, or lipases effective to selectively hydrolyze triglycerides.

### C. Immobilization of Enzymes

The enzymes used in the processes described herein may be immobilized, for which various materials and methods are known in the art; exemplary procedures are described below. Enzyme immobilization, in general, prolongs the useful life of the enzymes, simplifies purification of the products, and often enhances the catalytic activity of the enzyme. Materials onto which enzymes have been immobilized include silica, porous glass, Celite^{®}, diatomaceous earth, ion exchange resins, and various other polymeric substrates including polyamides, polypropylene, polyethylene glycol, polysaccharides such as cellulose, agarose, and alkyl modified agarose and chitin or chitosan. Polymeric substrates may be provided, for example, in the form of membranes, hollow fibers, or beads. In one embodiment, a membrane used for separation of fatty acids in the reaction could also be used for immobilization of enzymes. Immobilization is frequently accomplished by simple adsorption but may include covalent attachment, e.g. to amino or aldehyde modified silica.

Extensive descriptions of enzyme immobilization, including particular description of lipase and phospholipase immobilization, are available in the literature. A sampling of references includes K. Mosbach, ed., 1988; F.X. Malcata *et al.,* 1990, 1992; V.M. Balcao *et al.,* 1995; A.E. Ivanov *et al.,* 1997; G.F. Bickerstaff, ed., 1997; Bastida *et al.,* 1998; R Fernandez-Lafuente *et al.,* 1998; W. Cho *et al.,* 1999; U.T. Bornscheuer *et al.,* 2002; M.V. Ramachandra *et al.,* 2002; and D. Adlercreutz *et al.,* 2002; all cited above under "References". Enzymes are frequently provided commercially in immobilized form.

### IV. Hydrolyzed Product

Further described is a hydrolyzed product, obtained via an enzymatic hydrolysis reaction described herein, containing phospholipids and/or hydrolyzed phospholipids, monoglycerides, and diglycerides, and typically containing phospholipids and triglycerides. The hydrolyzed phospholipids may include lysophospholipids, phosphatidic acid, and/or lysophosphatidic acid. The hydrolyzed phospholipids are typically lysophospholipids, such as are produced by reaction of phospholipase A1 or A2.

The hydrolyzed product contains at least 0.5%, at least 1%, at least 2.5%, at least 5%, at least 7.5%, at least 10%, at least 15%, at least 20%, at least 25%, at least 30%, at least 35%, at least 40%, or at least 45% by weight mono/diglycerides. The mole percent of triglyceride converted to mono/diglycerides is preferably at least 25%, more preferably at least 50%, up to 75% or higher.

The products of the processes described herein are termed "hydrolyzed lecithin" or "hydrolyzed lecithin products" if their composition is in accordance with labeling standards for lecithins, which may vary by country. For example, according to the E322 labeling standard in Europe, "lecithins" are defmed as "mixtures of fractions of phospholipids which are obtained from animal or vegetable foodstuffs by physical processes"; they may also include "hydrolyzed substances which are obtained by the use of harmless and suitable enzymes". According to this labeling standard, a "lecithin" includes at least 60% acetone insoluble substances and has an acid value of less than 35 mg KOH/gram, and a "hydrolyzed lecithin" includes at least 56% acetone insoluble substances and has an acid value of less than 45 mg KOH/gram.

The hydrolyzed lecithin product may be derived from a lecithin starting material containing mixtures of phospholipids obtained from animal or vegetable foodstuffs by physical processes. The lecithin starting material may be obtained, for example, from water degumming or membrane degumming of crude soybean oil

The hydrolyzed product consists essentially of components of the starting material, which is derived directly from a vegetable oil, and hydrolyzed substances which are obtained from the starting material by the use of harmless and suitable enzymes, specifically, a lipase and, in most instances, a phospholipase, as described herein. The starting material is preferably a lecithin material, obtained by degumming of a vegetable oil, such as soybean oil. By "consists essentially of" is meant that the hydrolyzed product may contain, for example, water, but it does not contain lipid components other than the starting material components and hydrolyzed substances derived therefrom, as specified above. It does not contain residual enzyme activity.

### V. Exemplary Reaction Procedures

The following reactions are exemplary only and are not intended to limit the invention. These procedures typically refer to a "lecithin" starting material; however, as noted above, starting materials containing varying amounts of phospholipids and triglycerides may be used For example, a retentate from a membrane degumming process can include from about 10% to about 85% phospholipids, depending on the conditions of separation, as noted above.

### A. Hydrolysis in aprotic organic Solvent; Sequential Addition of Two Enzymes

A fluid lecithin obtained as the retentate from a membrane degumming process, as described, for example, in U.S. Patent No. 6,207,209, having an acetone insoluble (AI) level of 55%-80%, is combined with phospholipase A2, which may be immobilized, preferably in an amount of 0.001 to 0.2%, based on 60% AI. If necessary, the pH is adjusted to a level favorable to the enzyme, and CaCl₂ is added to activate the enzyme. Excess CaCl₂ may be used to efficiently sequester released fatty acids. Reaction is carried out at about 20-60°C for 1 to 24 hours. Immobilized enzyme is recovered, and drying provides a product containing lysolecithin, in an amount determined by reaction time and temperature and enzyme concentration.

To this product is added a lipase, which may be immobilized, preferably in an amount of 0.001 to 0.2%, based on 60% AI. If necessary, the pH is adjusted to a level favorable to the enzyme, and CaCl₂ is added Reaction is carried out at about 20-60°C for 1 to 24 hours. Immobilized enzyme is recovered, and drying provides a lecithin product containing lysolecithin and also containing mono/diglycerides, in an amount determined by reaction time and temperature and enzyme concentration.

In a preferred embodiment, the reaction is carried out in the presence of a membrane having a composition and pore size effective to selectively remove fatty acids from the reaction mixture.

Preferably, the lipase concentration and other parameters are effective to produce a hydrolyzed lecithin product containing at least 0.5%, at least 1 %, at least 2.5%, at least 5%, at least 7.5%, at least 10%, at least 15%, at least 20%, at least 25%, at least 30%, at least 35%, at least 40%, or at least 45% by weight mono/diglycerides. The mole percent of triglyceride converted to mono/diglycerides is preferably at least 25%, more preferably at least 50%, up to 75% or higher.

### B. Hydrolysis in aprotic organic Solvent; Simultaneous Addition of Two Enzymes

A fluid lecithin obtained as the retentate from a membrane degumming process, as described, for example, in U.S. Patent No. 6,207,209, having an acetone insoluble (AI) level of 55%-80%, is combined with phospholipase A2 and a triglyceride selective lipase, each of which may be immobilized, preferably each in an amount of 0.001 to 0.2%, based on 60% AI. If necessary, the pH is adjusted to a level favorable to the enzymes, and CaCl₂ is added Reaction is carried out at about 20-60°C for 1 to 24 hours. In a preferred embodiment, the reaction is carried out in the presence of a membrane having a composition and pore size effective to selectively remove fatty acids from the reaction mixture.

Immobilized enzyme is recovered, and drying provides a lecithin product containing lysolecithin and mono/diglycerides, each in an amount determined by reaction time and temperature and respective enzyme concentration. Preferably, the lipase concentration and other parameters are effective to produce a hydrolyzed lecithin product containing at least 0.5%, at least 1%, at least 2.5%, at least 5%, at least 7.5%, at least 10%, at least 15%, at least 20%, at least 25%, at least 30%, at least 35%, at least 40%, or at least 45% by weight mono/diglycerides. The mole percent of triglyceride converted to mono/diglycerides is preferably at least 25%, more preferably at least 50%, up to 75% or higher.

### C. Hydrolysis in aprotic organic Solvent; Single Enzyme

A fluid lecithin obtained as the retentate from a membrane degumming process, as described, for example, in U.S. Patent No. 6,207,209, having an acetone insoluble (AI) level of 20%-80%, is combined with a triglyceride selective lipase, which may be immobilized, in an amount of about 0.1 to 0.2% based on 60% AI. If necessary, the pH is adjusted to a level favorable to the enzyme, and CaCl₂ is added Excess CaCl₂ may be used to efficiently sequester released fatty acids. Reaction is carried out at about 20-60°C for 1 to 24 hours. In a preferred embodiment, the reaction is carried out in the presence of a membrane having a composition and pore size effective to selectively remove fatty acids from the reaction mixture.

Immobilized enzyme is recovered, and drying provides a lecithin product containing mono/diglycerides, in an amount determined by reaction time and temperature and respective enzyme concentration. Preferably, the lipase concentration and other parameters are effective to produce a hydrolyzed lecithin product containing at least 0.5%, at least 1%, at least 2.5%, at least 5%, at least 7.5%, at least 10%, at least 15%, at least 20%, at least 25%, at least 30%, at least 35%, at least 40%, or at least 45% by weight mono/diglycerides. The mole percent of triglyceride converted to mono/diglycerides is preferably at least 25%, more preferably at least 50%, up to 75% or higher.

### VI. Working Examples

The following examples are intended to illustrate and not to limit the invention.

### Immobilization of Enzymes

Immobilization of enzymes can be carried out as described, for example, in D. Adlercreutz *et al.,* 2002. Two conjugates, prepared as described below, were used in the following Examples.

Conjugate I: Lecitase®Ultra was immobilized on MP1000 (Membrana, Accurel Systems, Obemburg, Germany) as follows. MP1000 (3 g) was wetted using 25 ml ethanol (95%), and 2.5 ml Lecitase®Ultra (a Phospholipase A) in 350 ml 20 mM phosphate buffer (pH 7) was added. After 1h of stirring at 0°C, the immobilized enzyme was isolated by filtration and dried at reduced pressure.

Conjugate II: In a similar procedure, MP1000 (15 g) was wetted using 100 ml ethanol (95%), and 7.5 ml Lecitase®Ultra in 350 ml 20 mM phosphate buffer (pH 7) was added. After 1h of stirring at 0°C, the immobilized enzyme was isolated by filtration and dried at reduced pressure.

### Example I: Hydrolysis of Lecithin with Phospholipase A

A fluid lecithin retentate from a membrane degumming process, as described, for example, in patent No. 6,207,209, having an acetone insoluble (AI) level of 55%-80%, was recreated by dissolving 106.4 g lecithin (AI: 65.13, AV: 17.89 mg KOH/g), obtained by drying of such a retentate, and 14.08 g water in 564.5 g hexane.

This solution (60 ml) was combined with 200 mg of immobilized Lecitase® Ultra (conjugate I, prepared as described above.) Reaction was carried out at room temperature for 20 h, and the immobilized enzyme was recovered by filtration. Drying provided a product having an acid value of 35.1 mg KOH/g and the following composition (by acid value, P-NMR and GC): 17.8% fatty acids, 0.1% monoglycerides, 0.6% diglycerides, 26.2% triglycerides, 42.0% phospholipids and 2.5% lysophospholipids.

### Example II: Sequential Two-Enzyme Reaction

A fluid lecithin retentate from a membrane degumming process, as described, for example, in patent No. 6,207,209, having an acetone insoluble (AI) level of 55%-80%, was recreated by dissolving 106.4 g lecithin (AI: 65.13, AV: 17.89 mg KOH/g), obtained by drying of such a retentate, and 14.08 g water in 564.5 g hexane.

This solution (60 ml) was combined with 200 mg of immobilized Lecitase® Ultra (conjugate I, prepared as described above.) Reaction was carried out at room temperature for 20 h, and the immobilized enzyme was recovered by filtration.

To this filtrate 20 mg Lipozyme was added. Reaction was carried out at room temperature for 4 h, and the immobilized enzyme was recovered by filtration. Drying provided a product having an acid value of 62.5 mg KOH/g and the following composition (by acid value, P-NMR and GC): 31.5% fatty acids, 2.3% monoglycerides, 5.7% diglycerides, 9.4% triglycerides, 30.8% phospholipids and 8.4% lysophospholipids.

### Example III: Simultaneous Two-Enzyme Reaction

A fluid lecithin retentate from a membrane degumming process, as described, for example, in patent No. 6,207,209, having an acetone insoluble (AI) level of 55%-80%, was recreated by dissolving 106.4 g lecithin (AI: 65.13, AV: 17.89 mg KOH/g), obtained by drying of such a retentate, and 14.08 g water in 564.5 g hexane.

This solution (60 ml) was combined with 200 mg immobilized Lecitase® Ultra (conjugate II, prepared as described above) and 10 mg Lipozyme. Reaction is carried out at room temperature for 8 h, and the immobilized enzyme was recovered by filtration. Drying provided a product having an acid value of 54.3 mg KOH/g and the following composition (by acid value, P-NMR and GC): 27.3% fatty acids, 3.8% monoglycerides, 5.4% diglycerides, 3.1 % triglycerides, 44.6% phospholipids and 1.9% lysophospholipids.

### Example IV: Simultaneous Two-Enzyme Reaction

The procedure of Example III was repeated, using 100 mg of conjugate II instead of 200 mg. Drying provided a product having the following composition (by acid value, P-NMR and GC): 26% fatty acids, 3.6% monoglycerides, 5.2% diglycerides, 3.1 % triglycerides, 46% phospholipids and 1.1% lysophospholipids.

### Example V: Simultaneous Two-Enzyme Reaction

The procedure of Example III was repeated, using 400 mg of conjugate II instead of 200 mg. Drying provided a product having the following composition (by acid value, P-NMR and GC): 30.8% fatty acids, 3.9% monoglycerides, 4.3% diglycerides, 2.4% triglycerides, 43.6% phospholipids and 2.6% lysophospholipids.

### Example VI: Hydrolysis of Lecithin with a Lipase

A fluid lecithin retentate from a membrane degumming process, as described, for example, in patent No. 6,207,209, having an acetone insoluble (AI) level of 55%-80%, was recreated by dissolving 106.4 g lecithin (AI: 65.13, AV: 17.89 mg KOH/g), obtained by drying of such a retentate, and 14.08 g water in 564.5 g hexane.

This solution (60 ml) was combined with 10 mg Lipozyme. Reaction was carried out at room temperature for 20 h, and the immobilized enzyme was recovered by filtration. Drying provided a product having an acid value of 52.1 mg KOH/g and the following composition (by acid value, P-NMR and GC): 26.2% fatty acids, 4.0% monoglycerides, 5.3% diglycerides, 3.8% triglycerides, 47.4% phospholipids and 0.8% lysophospholipids.

Tables I and II below summarize the results of the above-described reactions. (PL = phospholipids; LPL = lysophospholipids; TG = triglycerides; DG = diglycerides; MG = monoglycerides; FA = fatty acids.) Table I shows the distribution of components in the hydrolyzed product, by weight percent. Table II shows the approximate molar ratio of triglycerides, diglycerides and monoglycerides (based on the molecular weight of a stearoyl glyceride) in the hydrolyzed product, relative to the sum of these three components. As can be seen, the mole percent of triglyceride converted to mono/diglycerides, in all Examples except I, is about 60% or higher, up to about 85%.

**Table I**

| **Example** | **Enzyme(s)** | **Product Acid Value** | **Product Components, wt %** | | | | | |
|---|---|---|---|---|---|---|---|---|
| | | | **PL** | **LPL** | **TG** | **DG** | **MG** | **FA** |
| I | Lecitase prep 200 mg | 35.1 | 42.0 | 2.5 | 26.2 | 0.6 | 0.1 | 17.8 |
| II | Lecitase prep 200 mg, followed by Lipozyme 20 mg | 62.5 | 30.8 | 8.4 | 9.4 | 5.7 | 2.3 | 31.5 |
| VI | Lipozyme 10 mg | 52.1 | 47.4 | 0.8 | 3.8 | 5.3 | 4.0 | 26.2 |
| IV | Lecitase prep 100 mg + Lipozyme 10 mg | n.d | 46.0 | 1.1 | 3.1 | 5.2 | 3.6 | 26.0 |
| III | Lecitase prep 200 mg + Lipozyme 10 mg | 54.3 | 44.6 | 1.9 | 3.1 | 5.4 | 3.8 | 27.3 |
| V | Lecitase prep 400 mg + Lipozyme 10 mg | n.d. | 43.6 | 2.6 | 2.4 | 4.3 | 3.9 | 30.8 |

**Table II**

| **Example** | **Enzyme(s)** | **TG/DG/MG Molar Ratio** | | |
|---|---|---|---|---|
| | | **Approx. mol% TG** | **Approx. mol% DG** | **Approx. mol% MG** |
| I | Lecitase prep 200 mg | 96% | 3% | 1% |
| II | Lecitase prep 200 mg, followed by Lipozyme 20 mg | 40% | 35% | 25% |
| VI | Lipozyme 10 mg | 18% | 36% | 46% |
| IV | Lecitase prep 100 mg + Lipozyme 10 mg | 16% | 38% | 46% |
| III | Lecitase prep 200 mg + Lipozyme 10 mg | 15% | 38% | 47% |
| V | Lecitase prep 400 mg + Lipozyme 10 mg | 13% | 34% | 53% |

### VII. Applications

The hydrolyzed lecithin products, prepared according to the present invention can be used in any application where lecithin, hydrolyzed lecithin, lecithin fortified with mono/diglycerides, or related products have been used. The hydrolyzed products of the invention can be formulated into such products according to methods known in the art for formulation of lecithin or hydrolyzed lecithin.

The hydrolyzed product can be used, for example, as an emulsifier, surfactant, stabilizer, releasing agent, wetting agent, dispersing agent, lubricant, viscosity control agent, crystallization agent, softening agent, emollient, anti-dusting agent, or high nutritional ingredient.

Various applications in which the hydrolyzed product can be used include food, feed, technical, cosmetic, and pharmaceutical/nutraceutical applications. Exemplary food applications include chocolate, chocolate derivatives, bakery, confectionary, icings, dairy products, cheese products, pasta products, margarine, shortening, fat mixtures, emulsions, spray oils, dressings, instantizing of cacao, milk, non dairy protein powders, release agents, soups, sauces, mayonnaises, dressings, meats, gravies, canned meats, meat analogues, bread improvers, beverages, energy drinks, snacks, desserts (such as ice cream and bars), meal improvers, bread improvers, chewing gum, colors, flavor mixes, emulsifier mixes, baby food, and antioxidants.

In a particular embodiment, hydrolyzed lecithin products prepared according to the invention, containing mono/diglycerides, are useful for improving antispattering properties in cooking products such as margarine or shortening. As provided herein, such products are preferably formed by hydrolysis of a lecithin starting material with a lipase, alone or in combination with a phospholipase.

Exemplary feed applications include emulsifiers and sources of high nutritional value in feed for, for example, fish, shrimp, calves (as milk replacer), pigs, sows, piglets, mink, poultry, and pets. Exemplary technical applications include use as a dispersing agent in, for example, paints, inks, coatings, magnetic tapes, and discs, as a softening agent in, for example, leather and textiles, as an emulsifier in, for example, crop protection and agrochemicals, or in lubricants, oils, adhesives, adsorbents, flocculants, corrosion inhibitors, ceramics, glass, detergents, metal processing, paper products, petroleum products, photocopying, photography, polymers, rubbers, and textiles. Exemplary cosmetic applications include use as a dispersing agent in lipstick and nail polish and as an emulsifier/stabilizer in shampoos, creams, and lotions. Exemplary pharmaceutical/nutraceutical applications include use as a natural source of phospholipids, such as phosphatidyl choline and vitamin E.

Further disclosed are methods of formulating a hydrolyzed lecithin product, as prepared by any of the invention methods described herein, in a product as described above, and particularly in a food product. Also disclosed is a product, especially a food product, containing a hydrolyzed lecithin product, as prepared by any of the invention methods described herein.

## Claims

1. A method of producing a hydrolyzed lecithin product, comprising hydrolyzed phospholipids, monoglycerides, and diglycerides, the method comprising
(a) contacting a lecithin material, which comprises a phospholipid component and a triglyceride component, with a first enzyme to create a reaction mixture in an organic solvent medium comprising an aprotic organic solvent and sufficient water to promote hydrolysis, wherein the first enzyme comprises a phospholipase or lipase which hydrolyzes the phospholipids;
(b) subsequently contacting the product of step (a), in an organic solvent medium containing sufficient water to promote hydrolysis, with a second enzyme that comprises a lipase which hydrolyzes the triglycerides and is different than the first enzyme; and
(c) obtaining the hydrolyzed lecithin product.

2. The method of claim 1, wherein the aprotic organic solvent comprises a hydrocarbon solvent.

3. The method of claim 2, wherein the aprotic organic solvent is hexane.

4. The method of claim 1, wherein steps (a) and (b) are carried out in the presence of a membrane that separates the hydrolyzed phospholipids, monoglycerides, and diglycerides from released fatty acids.

5. The method of claim 1, wherein the lecithin material is comprised of at least 40% of the phospholipid component.

6. The method of claim 1, wherein the lecithin material comprises at least 60% acetone insoluble material.

7. The method of claim 1, wherein the lecithin material comprises a retentate from a vegetable oil membrane degumming process.

8. The method of claim 1, wherein the hydrolyzed lecithin product comprises at least 56% acetone insoluble materials and has an acid value of less than 45 mg KOH/gram.

9. The method of claim 1, wherein the hydrolyzed lecithin product comprises at least 60% acetone insoluble materials.

10. The method of claim 1, wherein the first enzyme is phospholipase A1, phospholipase A2, or a combination thereof.

11. The method of claim 1, wherein the first enzyme is phospholipase A2.

12. The method of claim 1, wherein the first enzyme is phospholipase C.

13. The method of claim 1, wherein the first enzyme is phospholipase D.

14. The method of claim 5, further comprising, prior to the contacting step (b), reacting the product of step (a) with phospholipase A1, phospholipase A2, or a combination thereof.

15. The method of claim 1, wherein the second enzyme selectively hydrolyzes the triglycerides.

16. The method of claim 1, wherein the second enzyme is a lipase.

17. The method of claim 1, wherein the second enzyme is Lipozyme.

18. A method of producing a hydrolyzed lecithin product comprising hydrolyzed phospholipids, monoglycerides, and diglycerides, the method comprising:
contacting a lecithin material, which comprises a phospholipid component and a triglyceride component with first and second enzymes in a reaction mixture containing an aprotic organic solvent and sufficient water to promote hydrolysis; wherein, the first enzyme comprises a phospholipase or lipase which hydrolyzes the phospholipids, and the second enzyme comprises a lipase which hydrolyzes the triglycerides and is different than the first enzyme; and obtaining the hydrolyzed lecithin product from the reaction mixture.

19. The method of claim 18, wherein the contacting is carried out in the presence of a membrane that separates the hydrolyzed phospholipids, monoglycerides, and diglycerides from released fatty acids.

20. The method of claim 18, wherein the aprotic organic solvent comprises a hydrocarbon solvent.

21. The method of claim 18, wherein the aprotic organic solvent is hexane.

22. The method of claim 18, wherein the lecithin material is comprised of at least 40% of the phospholipid component.

23. The method of claim 18, wherein the lecithin material comprises at least 60% acetone insoluble material.

24. The method of claim 18, wherein the lecithin material comprises a retentate from a vegetable oil membrane degumming process.

25. The method of claim 18, wherein the hydrolyzed lecithin product comprises at least 56% acetone insoluble materials and has an acid value of less than 45 mg KOH/gram.

26. The method of claim 18, wherein the hydrolyzed lecithin product comprises at least 60% acetone insoluble materials.

27. The method of claim 18, wherein the first enzyme is phospholipase A1, phospholipase A2, or a combination thereof.

28. The method of claim 18, wherein the first enzyme is phospholipase A2.

29. The method of claim 18, wherein the first enzyme is phospholipase C.

30. The method of claim 18, wherein the first enzyme is phospholipase D.

31. The method of claim 18, wherein the second enzyme selectively hydrolyzes the triglycerides.

32. The method of claim 18, wherein the second enzyme is a lipase.

33. The method of claim 18, wherein the second enzyme is Lipozyme.

34. The method of claim 18, wherein the lecithin material is contacted with the first and second enzymes concurrently.

35. A method of producing a product comprising phospholipids, monoglycerides, and diglycerides by enzymatic hydrolysis, the method comprising:
contacting a lecithin material, which comprises a phospholipid component and a triglyceride component, with a lipase in the absence of a phospholipase to create a reaction mixture in an organic solvent medium comprising an aprotic organic solvent and sufficient water to promote hydrolysis, to selectively hydrolyze the triglycerides; and obtaining the hydrolyzed lecithin product from the reaction mixture, wherein the lecithin material is a retentate from a vegetable oil membrane degumming process.

36. The method of claim 35, wherein the lecithin material is comprised of at least 40% of the phospholipid component.

37. The method of claim 35, wherein the lecithin material comprises at least 60% acetone insoluble material.

38. The method of claim 35, wherein the hydrolyzed lecithin product comprises at least 56% acetone insoluble materials and has an acid value of less than 45 mg KOH/gram.

39. The method of claim 35, wherein the hydrolyzed lecithin product comprises at least 60% acetone insoluble materials.

40. The method of claim 35, wherein the lipase is Lipozyme.

41. A method of producing a product comprising phospholipids, monoglycerides, and diglycerides by enzymatic hydrolysis, the method comprising:
contacting a lecithin material, which comprises a phospholipids component and a triglyceride component, with a lipase in the absence of a phospholipase to create a reaction mixture in an organic solvent medium comprising an aprotic organic solvent and sufficient water to promote hydrolysis, wherein the lipase selectively hydrolyzes the triglycerides; and
obtaining the hydrolyzed lecithin product from the reaction mixture, wherein the contacting is carried out in the presence of a membrane effective to separate the phospholipids, monoglycerides, and diglycerides from released fatty acids.

42. The method of claim 41, wherein the lecithin material is comprised of at least 40% of the phospholipid component.

43. The method of claim 41, wherein the lecithin material comprises at least 60% acetone insoluble material.

44. The method of claim 41, wherein the hydrolyzed lecithin product comprises at least 56% acetone insoluble materials and has an acid value of less than 45 mg KOH/gram.

45. The method of claim 41, wherein the hydrolyzed lecithin product comprises at least 60% acetone insoluble materials.

46. The method of claim 41, wherein the lipase is Lipozyme.

47. The method of claim 7, wherein the phospholipid component composes 50% to 85% of the retentate.

48. The method of claim 24, wherein the phospholipid component composes 50% to 85% of the retentate.

49. The method of claim 35, wherein the phospholipid component composes 50% to 85% of the retentate.

## Patentansprüche

1. Verfahren zur Herstellung eines hydrolysierten Lecithinprodukts, umfassend hydrolysierte Phospholipide, Monoglyceride und Diglyceride, wobei das Verfahren umfasst:
(a) das Inkontaktbringen einer Lecithinsubstanz, die einen Phospholipidbestandteil und einen Triglyceridbestandteil umfasst, mit einem ersten Enzym, um eine Reaktionsmischung in einem organischen Lösungsmittelmedium zu erstellen, umfassend ein aprotisches organisches Lösungsmittel und ausreichend Wasser, um Hydrolyse zu begünstigen, wobei das erste Enzym eine Phospholipase oder Lipase umfasst, die die Phospholipide hydrolysiert;
(b) das anschließende Inkontaktbringen des Produkts aus Schritt (a) in einem organischen Lösungsmittelmedium, das ausreichend Wasser, um Hydrolyse zu fördern, enthält, mit einem zweiten Enzym, das eine Lipase umfasst, die die Triglyceride hydrolysiert und ein anderes ist als das erste Enzym; und
(c) das Gewinnen des hydrolysierten Lecithinprodukts.

2. Verfahren nach Anspruch 1, wobei das aprotische organische Lösungsmittel ein Hydrocarbonlösungsmittel umfasst.

3. Verfahren nach Anspruch 2, wobei das aprotische organische Lösungsmittel Hexan ist.

4. Verfahren nach Anspruch 1, wobei die Schritte (a) und (b) in Gegenwart einer Membran durchgeführt werden, die die hydrolysierten Phospholipide, Monoglyceride und Diglyceride von freigesetzten Fettsäuren trennt.

5. Verfahren nach Anspruch 1, wobei die Lecithinsubstanz aus mindestens 40% des Phospholipidbestandteils besteht.

6. Verfahren nach Anspruch 1, wobei die Lecithinsubstanz mindestens 60% Aceton-unlösliche Substanzen umfasst.

7. Verfahren nach Anspruch 1, wobei das Lecithinmaterial ein Retentat eines Pflanzenöl-Membran-Degummierungsprozesses umfasst.

8. Verfahren nach Anspruch 1, wobei das hydrolysierte Lecithinprodukt mindestens 56% Aceton-unlösliche Substanzen umfasst und einen Säurewert von weniger als 45 mg KOH/g hat.

9. Verfahren nach Anspruch 1, wobei das hydrolysierte Lecithinprodukt mindestens 60% Aceton-unlösliche Substanzen umfasst.

10. Verfahren nach Anspruch 1, wobei das erste Enzym Phospholipase A1, Phospholipase A2 oder eine Kombination davon ist.

11. Verfahren nach Anspruch 1, wobei das erste Enzym Phospholipase A2 ist.

12. Verfahren nach Anspruch 1, wobei das erste Enzym Phospholipase C ist.

13. Verfahren nach Anspruch 1, wobei das erste Enzym Phospholipase D ist.

14. Verfahren nach Anspruch 5, das zudem, vor dem Schritt des Inkontaktbringens (b), das Reagieren des Produkts aus Schritt (a) mit Phospholipase A1, Phospholipase A2 oder einer Kombination davon umfasst.

15. Verfahren nach Anspruch 1, wobei das zweite Enzym selektiv die Triglyceride hydrolysiert.

16. Verfahren nach Anspruch 1, wobei das zweite Enzym eine Lipase ist.

17. Verfahren nach Anspruch 1, wobei das zweite Enzym Lipozym ist.

18. Verfahren zur Herstellung eines hydrolysierten Lecithinprodukts umfassend hydrolysierte Phospholipide, Monoglyceride und Diglyceride, wobei das Verfahren umfasst: das Inkontaktbringen einer Lecithinsubstanz, die einen Phospholipidbestandteil und einen Triglyceridbestandteil umfasst, mit ersten und zweiten Enzymen in einer Reaktionsmischung, die ein aprotisches organisches Lösungsmittel und ausreichend Wasser, um Hydrolyse zu begünstigen, enthält; wobei das erste Enzym eine Phospholipase oder Lipase, die die Phospholipide hydrolysiert, umfasst, und das zweite Enzym eine Lipase umfasst, die die Triglyceride hydrolysiert und ein anderes ist als das erste Enzym; und das Gewinnen des hydrolysierten Lecithinprodukts aus der Reaktionsmischung.

19. Verfahren nach Anspruch 18, wobei das Inkontaktbringen in Gegenwart einer Membran durchgeführt wird, die die hydrolysierten Phospholipide, Monoglyceride und Diglyceride von freigesetzten Fettsäuren separiert.

20. Verfahren nach Anspruch 18, wobei das aprotische organische Lösungsmittel ein Hydrocarbonlösungsmittel umfasst.

21. Verfahren nach Anspruch 18, wobei das aprotische organische Lösungsmittel Hexan ist.

22. Verfahren nach Anspruch 18, wobei die Lecithinsubstanz aus mindestens 40% des Phospholipidbestandteils besteht.

23. Verfahren nach Anspruch 18, wobei das Lecithinmaterial mindestens 60% Aceton-unlösliche Substanzen umfasst.

24. Verfahren nach Anspruch 18, wobei das Lecithinmaterial ein Retentat eines Pflanzenöl-Membran-Degummierungsprozesses umfasst.

25. Verfahren nach Anspruch 18, wobei das hydrolysierte Lecithinprodukt mindestens 56% Aceton-unlösliche Substanzen umfasst und einen Säurewert von weniger als 45 mg KOH/g hat.

26. Verfahren nach Anspruch 18, wobei das hydrolysierte Lecithinprodukt mindestens 60% Aceton-unlösliche Substanzen umfasst.

27. Verfahren nach Anspruch 18, wobei das erste Enzym Phospholipase A1, Phospholipase A2 oder eine Kombination davon ist.

28. Verfahren nach Anspruch 18, wobei das erste Enzym Phospholipase A2 ist.

29. Verfahren nach Anspruch 18, wobei das erste Enzym Phospholipase C ist.

30. Verfahren nach Anspruch 18, wobei das erste Enzym Phospholipase D ist.

31. Verfahren nach Anspruch 18, wobei das zweite Enzym selektiv die Triglyceride hydrolysiert.

32. Verfahren nach Anspruch 18, wobei das zweite Enzym eine Lipase ist.

33. Verfahren nach Anspruch 18, wobei das zweite Enzym Lipozym ist.

34. Verfahren nach Anspruch 18, wobei die Lecithinsubstanz mit dem ersten und zweiten Enzym gleichzeitig in Kontakt gebracht wird.

35. Verfahren zur Herstellung eines Produkts umfassend Phospholipide, Monoglyceride und Diglyceride durch enzymatische Hydrolyse, wobei das Verfahren umfasst:
das Inkontaktbringen einer Lecithinsubstanz, die einen Phospholipidbestandteil und einen Triglyceridbestandteil umfasst, mit einer Lipase in Abwesenheit einer Phospholipase, um eine Reaktionsmischung in einem organischen Lösungsmittelmedium zu erstellen, umfassend ein aprotisches organisches Lösungsmittel und ausreichend Wasser, um Hydrolyse zu begünstigen, um selektiv die Triglyceride zu hydrolysieren; und das Gewinnen des hydrolysierten Lecithinprodukts aus der Reaktionsmischung, wobei die Lecithinsubstanz ein Retentat eines Pflanzenöl-Membran-Degummierungsprozesses ist.

36. Verfahren nach Anspruch 35, wobei die Lecithinsubstanz aus mindestens 40% des Phospholipidbestandteils besteht.

37. Verfahren nach Anspruch 35, wobei die Lecithinsubstanz mindestens 60% Aceton-unlösliche Substanzen umfasst.

38. Verfahren nach Anspruch 35, wobei das hydrolysierte Lecithinprodukt mindestens 56% Aceton-unlösliche Substanzen umfasst und einen Säurewert von weniger als 45 mg KOH/g hat.

39. Verfahren nach Anspruch 35, wobei das hydrolysierte Lecithinprodukt mindestens 60% Aceton-unlösliche Substanzen umfasst.

40. Verfahren nach Anspruch 35, wobei die Lipase Lipozym ist.

41. Verfahren zur Herstellung eines Produkts, umfassend Phospholipide, Monoglyceride und Diglyceride durch enzymatische Hydrolyse, wobei das Verfahren umfasst:
das Inkontaktbringen einer Lecithinsubstanz, die einen Phospholipidbestandteil und einen Triglyceridbestandteil umfasst, mit einer Lipase in Abwesenheit einer Phospholipase, um eine Reaktionsmischung in einem organischen Lösungsmittelmedium zu erstellen, umfassend ein aprotisches organisches Lösungsmittel und ausreichend Wasser, um Hydrolyse zu begünstigen, wobei die Lipase selektiv die Triglyceride hydrolysiert; und das Gewinnen des hydrolysierten Lecithinprodukts aus der Reaktionsmischung, wobei das Inkontaktbringen in Gegenwart einer Membran durchgeführt wird, die wirksam ist, um Phospholipide, Monoglyceride und Diglyceride von freigesetzten Fettsäuren zu trennen.

42. Verfahren nach Anspruch 41, wobei die Lecithinsubstanz aus mindestens 40% des Phospholipidbestandteils besteht.

43. Verfahren nach Anspruch 41, wobei die Lecithinsubstanz mindestens 60% Aceton-unlösliche Substanzen umfasst.

44. Verfahren nach Anspruch 41, wobei das hydrolysierte Lecithinprodukt mindestens 56% Aceton-unlösliche Substanzen umfasst und einen Säurewert von weniger als 45 mg KOH/g hat.

45. Verfahren nach Anspruch 41, wobei das hydrolysierte Lecithinprodukt mindestens 60% Aceton-unlösliche Substanzen umfasst.

46. Verfahren nach Anspruch 41, wobei die Lipase Lipozym ist.

47. Verfahren nach Anspruch 7, wobei die Phospholipidkomponente 50% bis 85% des Retentats bildet.

48. Verfahren nach Anspruch 24, wobei die Phospholipidkomponente 50% bis 85% des Retentats bildet.

49. Verfahren nach Anspruch 35, wobei die Phospholipidkomponente 50% bis 85% des Retentats bildet.

## Revendications

1. Méthode de production d'un produit de lécithine hydrolysé, comprenant des phospholipides hydrolysés, des monoglycérides et des diglycérides, la méthode comprenant
(a) la mise en contact d'une substance de lécithine, qui comprend un composant phospholipide et un composant triglycéride, avec une première enzyme pour créer un mélange réactionnel dans un milieu de solvant organique comprenant un solvant organique aprotique et suffisamment d'eau pour promouvoir l'hydrolyse, dans laquelle la première enzyme comprend une phospholipase ou une lipase qui hydrolyse les phospholipides ;
(b) la mise en contact ensuite du produit de l'étape (a), dans un milieu de solvant organique contenant suffisamment d'eau pour promouvoir l'hydrolyse, avec une seconde enzyme qui comprend une lipase qui hydrolyse les triglycérides et qui est différente de la première enzyme ; et
(c) l'obtention du produit de lécithine hydrolysé.

2. Méthode selon la revendication 1, dans laquelle le solvant organique aprotique comprend un solvant hydrocarboné.

3. Méthode selon la revendication 2, dans laquelle le solvant organique aprotique est l'hexane.

4. Méthode selon la revendication 1, dans laquelle les étapes (a) et (b) sont réalisée en présence d'une membrane qui sépare les phospholipides hydrolysés, les monoglycérides et les diglycérides des acides gras libérés.

5. Méthode selon la revendication 1, dans laquelle la substance de lécithine comprend au moins 40 % de composant phospholipide.

6. Méthode selon la revendication 1, dans laquelle la substance de lécithine comprend au moins 60 % d'une substance insoluble dans l'acétone.

7. Méthode selon la revendication 1, dans laquelle la substance de lécithine comprend un rétentat provenant d'un procédé de démucilagination d'une membrane d'huile végétale.

8. Méthode selon la revendication 1, dans laquelle le produit de lécithine hydrolysé comprend au moins 56 % de substances insolubles dans l'acétone et a un indice d'acidité inférieur à 45 mg de KOH/g.

9. Méthode selon la revendication 1, dans laquelle le produit de lécithine hydrolysé comprend au moins 60 % de substances insolubles dans l'acétone.

10. Méthode selon la revendication 1, dans laquelle la première enzyme est la phospholipase A1, la phospholipase A2 ou une combinaison de celles-ci.

11. Méthode selon la revendication 1, dans laquelle la première enzyme est la phospholipase A2.

12. Méthode selon la revendication 1, dans laquelle la première enzyme est la phospholipase C.

13. Méthode selon la revendication 1, dans laquelle la première enzyme est la phospholipase D.

14. Méthode selon la revendication 5, comprenant en outre, avant l'étape de mise en contact (b), la réaction du produit de l'étape (a) avec la phospholipase A1, la phospholipase A2, ou une combinaison de celles-ci.

15. Méthode selon la revendication 1, dans laquelle la seconde enzyme hydrolyse sélectivement les triglycérides.

16. Méthode selon la revendication 1, dans laquelle la seconde enzyme est une lipase.

17. Méthode selon la revendication 1, dans laquelle la seconde enzyme est le Lipozyme.

18. Méthode de production d'un produit de lécithine hydrolysé comprenant des phospholipides hydrolysés, des monoglycérides et des diglycérides, la méthode comprenant : la mise en contact d'une substance de lécithine, qui comprend un composant phospholipide et un composant triglycéride avec une première et une seconde enzyme dans un milieu réactionnel contenant un solvant organique aprotique et suffisamment d'eau pour promouvoir l'hydrolyse ; dans laquelle la première enzyme comprend une phospholipase ou une lipase qui hydrolyse les phospholipides, et la seconde enzyme comprend une lipase qui hydrolyse les triglycérides et est différente de la première enzyme ; et l'obtention du produit de lécithine hydrolysé à partir du mélange réactionnel.

19. Méthode selon la revendication 18, dans laquelle la mise en contact est réalisée en présence d'une membrane qui sépare les phospholipides hydrolysés, les monoglycérides et les diglycérides des acides gras libérés.

20. Méthode selon la revendication 18, dans laquelle le solvant organique aproptique comprend un solvant hydrocarboné.

21. Méthode selon la revendication 18, dans laquelle le solvant organique aprotique est l'hexane.

22. Méthode selon la revendication 18, dans laquelle la substance de lécithine comprend au moins 40 % du composant phospholipide.

23. Méthode selon la revendication 18, dans laquelle la substance de lécithine comprend au moins 60 % d'une substance insoluble dans l'acétone.

24. Méthode selon la revendication 18, dans laquelle la substance de lécithine comprend un rétentat provenant d'un procédé de démucilagination d'une membrane d'huile végétale.

25. Méthode selon la revendication 18, dans laquelle le produit de lécithine hydrolysé comprend au moins 56 % de substances insolubles dans l'acétone et a un indice d'acidité inférieur à 45 mg de KOH/g.

26. Méthode selon la revendication 18, dans laquelle le produit de lécithine hydrolysé comprend au moins 60 % de substances insolubles dans l'acétone.

27. Méthode selon la revendication 18, dans laquelle la première enzyme est la phospholipase A1, la phospholipase A2, ou une combinaison de celles-ci.

28. Méthode selon la revendication 18, dans laquelle la première enzyme est la phospholipase A2.

29. Méthode selon la revendication 18, dans laquelle la première enzyme est la phospholipase C.

30. Méthode selon la revendication 18, dans laquelle la première enzyme est la phospholipase D.

31. Méthode selon la revendication 18, dans laquelle la seconde enzyme hydrolyse sélectivement les triglycérides.

32. Méthode selon la revendication 18, dans laquelle la seconde enzyme est une lipase.

33. Méthode selon la revendication 18, dans laquelle la seconde enzyme est le Lipozyme.

34. Méthode selon la revendication 18, dans laquelle la substance de lécithine est mise en contact simultanément avec la première et la seconde enzyme.

35. Méthode de production d'un produit comprenant des phospholipides, des monoglycérides et des diglycérides par hydrolyse enzymatique, la méthode comprenant :
la mise en contact d'une substance de lécithine, qui comprend un composant phospholipide et un composant triglycéride, avec une lipase en l'absence d'une phospholipase pour créer un mélange réactionnel dans un milieu de solvant organique comprenant un solvant organique aprotique et suffisamment d'eau pour promouvoir l'hydrolyse afin d'hydrolyser sélectivement les triglycérides ; et
l'obtention du produit de lécithine hydrolysé à partir du mélange réactionnel, dans laquelle la substance de lécithine est un rétentat provenant d'un procédé de démucilagination d'une membrane d'huile végétale.

36. Méthode selon la revendication 35, dans laquelle la substance de lécithine comprend au moins 40 % du composant phospholipide.

37. Méthode selon la revendication 35, dans laquelle la substance de lécithine comprend au moins 60 % de substance insoluble dans l'acétone.

38. Méthode selon la revendication 35, dans laquelle le produit de lécithine hydrolysé comprend au moins 56 % de substances insolubles dans l'acétone et a un indice d'acidité inférieur à 45 mg de KOH/g.

39. Méthode selon la revendication 35, dans laquelle le produit de lécithine hydrolysé comprend au moins 60 % de substances insolubles dans l'acétone.

40. Méthode selon la revendication 35, dans laquelle la lipase est le Lipozyme.

41. Méthode de production d'un produit comprenant des phospholipides, des monoglycérides et des diglycérides par hydrolyse enzymatique, la méthode comprenant :
la mise en contact d'une substance de lécithine, qui comprend un composant phospholipide et un composant triglycéride, avec une lipase en l'absence d'une phospholipase pour créer un mélange réactionnel dans un milieu de solvant organique comprenant un solvant organique aprotique et suffisamment d'eau pour promouvoir l'hydrolyse, dans laquelle la lipase hydrolyse sélectivement les triglycérides ; et
l'obtention du produit de lécithine hydrolysé à partir du mélange réactionnel, dans laquelle la mise en contact est réalisée en présence d'une membrane efficace pour séparer les phospholipides, les monoglycérides et les diglycérides des acides gras libérés.

42. Méthode selon la revendication 41, dans laquelle la substance de lécithine comprend au moins 40 % du composant phospholipide.

43. Méthode selon la revendication 41, dans laquelle la substance de lécithine comprend au moins 60 % de substances insolubles dans l'acétone.

44. Méthode selon la revendication 41, dans laquelle le produit de lécithine hydrolysé comprend au moins 56 % de substances insolubles dans l'acétone et a un indice d'acidité inférieur à 45 mg de KOH/g.

45. Méthode selon la revendication 41, dans laquelle le produit de lécithine hydrolysé comprend au moins 60 % de substances insolubles dans l'acétone.

46. Méthode selon la revendication 41, dans laquelle la lipase est le Lipozyme.

47. Méthode selon la revendication 7, dans laquelle le composant phospholipide représente de 50 % à 85 % du rétentat.

48. Méthode selon la revendication 24, dans laquelle le composant phospholipide représente de 50 % à 85 % du rétentat.

49. Méthode selon la revendication 35, dans laquelle le composant phospholipide représente de 50 % à 85 % du rétentat.
